# EUROPÄISCHE PATENTSCHRIFT

(11) **EP 3 952 800 B1**
(45) Veröffentlichungstag und Bekanntmachung des Hinweises auf die Patenterteilung: **12.07.2023**
(21) Anmeldenummer: 20714652.3
(22) Anmeldetag: 03.04.2020
(51) Int. Cl.: A61F 5/01, A61F 5/058, A61F 5/10

(54) **DAUMENORTHESE UND SET MIT MEHREREN DAUMENORTHESEN**
THUMB ORTHOTIC AND SET COMPRISING SEVERAL THUMB ORTHOTICS
ORTHÈSE DE POUCE ET ENSEMBLE POURVU D'UNE PLURALITÉ D'ORTHÈSES

(30) Priorität: 12.04.2019 EP 19169043
(43) Veröffentlichungstag der Anmeldung: 16.02.2022
(73) Patentinhaber: CHW-Technik GmbH, 37115 Duderstadt (DE); Talke, Martin, 13595 Berlin (DE); Roller, Matthias, 72336 Balingen (DE)
(72) Erfinder: WAGNER, Helmut, 37115 Duderstadt (DE); TALKE, Martin, 13595 Berlin (DE); ROLLER, Matthias, 72336 Balingen (DE)
(74) Vertreter: REHBERG HÜPPE + PARTNER
(86) Internationale Anmeldenummer: PCT/EP2020/059547
(87) Internationale Veröffentlichungsnummer: WO 2020/207916

(56) Entgegenhaltungen:
- JP-A- 2011 194 187
- JP-A- 2017 080 354
- US-A1- 2012 179 081
- US-A1- 2016 296 359

## Beschreibung

### TECHNISCHES GEBIET DER ERFINDUNG

Daumenorthesen finden insbesondere dann Einsatz, wenn gewährleistet werden soll, dass ein Daumen einer Hand eine Grundstellung einnimmt, in der der Daumen zumindest teilweise von dem Zeigefinger der Hand abgespreizt ist. Beispielsweise kann einer Neigung eines Patienten mit Rheuma, zur Vermeidung von Schmerzen den Daumen an den Zeigefinger anzulegen, durch den Einsatz einer Daumenorthese Rechnung getragen werden. Andere nicht beschränkende Beispiele, in denen der Einsatz einer Daumenorthese sinnvoll ist, kann die Therapie chronischer, posttraumatischer oder postoperativer Reizzustände, pathologischer Zustände im Bereich der zugeordneten Gelenke, der sogenannten Rhizarthrose des Daumens (Arthrose bzw. degenerativer Knorpelschwund im Bereich des Daumensattelgelenks) oder eines sogenannten Skidaumens, bei dem es nach einem Wegknicken des Daumens im Grundgelenk nach außen infolge eines Sturzes zu einer Seitenbandruptur des Daumens kommt. Möglich ist bspw. auch der Einsatz der Daumenorthese für eine Schonung der Daumengelenke beispielsweise vor Überanstrengung oder (erneuter) Verletzung, als präventive Maßnahme oder zur Ruhigstellung eines Karpometakarpalgelenks. Neben der Gewährleistung der Grundstellung des Daumens werden an Daumenorthesen auch die Anforderungen gestellt, dass diese einen hohen Tragekomfort gewährleisten sollen, möglichst einen Pinzettengriff ermöglichen soll, bei dem die Spitzen von Daumen und Zeigefinger zum Ergreifen eines Gegenstands pinzettenartig aneinander angenähert werden können und die Orthese ein optisch ansprechendes und möglichst unauffälliges Erscheinungsbild aufweisen soll.

Die vorliegende Erfindung betrifft eine Daumenorthese und ein Set mit mehreren derartigen Daumenorthesen.

### STAND DER TECHNIK

DE 195 11 116 A1 offenbart eine Daumenorthese zur Korrektur von Fehlstellungen des Daumens im Sattelgelenk. Die Daumenorthese weist einen ringartigen Spreizkörper auf. Der Daumen wird durch den ringförmigen Spreizkörper hindurchgesteckt, so dass der distale Endbereich des ringförmigen Spreizkörpers in dem Übergangsbereich der Hand vom Daumen zum Zeigefinger (der im Folgenden auch als "Daumenbogen" bezeichnet wird) anliegt, während der proximale Endbereich des ringförmigen Spreizkörpers ungefähr im Bereich des Sattelgelenks des Daumens an der Hand anliegt. In dieser Stellung wird der ringförmige Spreizkörper über ein Halteband gesichert. Das Halteband ist in einem Endbereich auf der Innenseite der Hand an dem ringförmigen Spreizkörper befestigt, erstreckt sich im Bereich des Handgelenks um die Hand und ist in dem anderen Endbereich auf der Außenseite der Hand wieder mit dem ringförmigen Spreizkörper verbunden. Der ringförmige Spreizkörper besteht aus einem thermoplastischen Kunststoff, der mit einer Pelotte ausgestattet sein kann. Das Halteband kann elastisch sein und ein Spannen des Haltebands kann mittels eines Klettverschlusses ermöglicht werden.

Auch DE 10 2016 010 135 A1 offenbart eine Daumenorthese mit einem ringförmigen Spreizkörper, durch welchen der Daumen hindurchgesteckt wird, und mit einem die Hand oberhalb des Handgelenks umschlingenden Halteband.

WO 2013/160478 A1 offenbart eine Orthese, mittels welcher eine Immobilisierung des Daumensattel- und -grundgelenks erfolgen soll. Die Orthese besteht aus einem steifen Mittelhandabschnitt sowie einem steifen Daumenabschnitt. Der Mittelhandabschnitt ist als komplex geformter Kreisring ausgebildet und soll die Hand im Bereich der Handinnenfläche teilweise umschließen, so dass über die Wechselwirkung des Mittelhandabschnitts mit der Hand im Bereich der Handinnenfläche eine Position und Ausrichtung der Orthese vorgegeben werden kann. Der steife Daumenabschnitt kragt von einem Endbereich des Mittelhandabschnitts aus und verfügt über einen ringförmigen oder ringsegmentförmigen Daumenhalteabschnitt, in den der Daumen der Hand so eingelegt werden kann, dass der Daumen gegen eine Bewegung in Richtung auf die Handfläche abstützt ist. Hierdurch wird eine Grundstellung des Daumens gewährleistet, in der der Daumen und der Zeigefinger der Hand gegenübergestellt sind und der Zeigefinger in Richtung auf den Daumen in Anlage an diesen und von dem Daumen weg bewegt werden kann. Eine individuelle Anpassung der Orthese an die Hände verschiedener Benutzer soll durch eine plastische Verformung der Orthese (insbesondere unter Wärmeeinwirkung) möglich sein. Die Orthese kann aus Metall, insbesondere Aluminium oder Kunststoff oder einem mit Kunststoff umgebenen Metallkern, insbesondere einem Aluminiumkern, hergestellt sein. Die Orthese kann im Kontaktbereich mit der Hand eine Polsterung (insbesondere ein textiles Polster, ein Schaumstoffpolster, ein Gelpolster oder eines Luftpolster) aufweisen. Eine zusätzliche Fixierung der Orthese kann über ein Band mit einem geeigneten Verschlusselement erfolgen. Bei dem eingesetzten Kunststoff handelt es sich insbesondere um ein thermoplastisches Elastomer.

Weitere Daumenorthesen sind aus
- der Druckschrift DE 20 2006 011 664 U1,
- dem Produkt "Push Ortho Daumenorthese CMC" (vgl. www.careshop.de),
- einer Daumenschiene des Unternehmens BORT (vgl. www.bort.com) und
- einer Orthese "Rhizo-Ring" des Unternehmens Sporlastic (vgl. www. sporlastic.de)
bekannt, wobei auch hier eine Fixierung eines ringartigen Spreizkörpers über Haltebänder, die die Hand im Bereich des Handgelenks umschlingen, erfolgt oder die Orthese als steifer "Handschuh" unter Freilassung eines Teils der Hand und des Zeigefingers, Mittelfingers, Ringfingers und kleinen Fingers ausgebildet ist.

JP 2017-080354 A kritisiert aus dem Stand der Technik bekannte Orthesen zur Fixierung eines Daumens gemäß den Druckschriften JP 2012-057286 A und JP 2005-074078 A dahingehend, dass diese einen komplexen Aufbau mit mehreren Strukturelementen und Bestandteilen aus unterschiedlichen Materialien aufweisen, das Anlegen, Halten und Fixieren der Orthese kompliziert ist und die Haltefunktion und/oder Fixierfunktion nicht hinreichend erfüllt werden kann. Dabei findet gemäß JP 2012-057286 A eine Art abgeschnittener Handschuh Einsatz, aus dem sich die Finger mit einem Teil der Hand frei heraus erstrecken können, während der Daumen durch eine endseitig offene Hülse für den Durchtritt der Daumenspitze fixiert ist. Der Handschuh kann dabei mittels eines Spannriemens um die Hand gespannt werden und verfügt über mehrere komplexe, Y-förmige Verstärkungselemente. Hingegen verfügt die aus JP 2005-074078 A bekannte Daumenorthese über eine starre, hülsenartige Daumenaufnahme, deren proximaler Endbereich erweitert ist und sich bis zum Daumenbogen und mit einem Befestigungsabschnitt bis zu dem Handgelenk erstreckt. In dem Befestigungsbereich der Daumenaufnahme ist ein Spanngurt befestigt, welcher das Handgelenk umschlingt, womit die Orthese an der Hand des Benutzers fixiert werden kann. Um diesen komplexen Aufbau mit einer Vielzahl von Materialien und Strukturelementen und einer Erschwernis des Anlegens sowie Fixierens der Orthese zu vermeiden, schlägt JP 2017-080354 A vor, zur Behandlung einer Tendinitis den Daumen zu fixieren über eine pferdesattelförmige Orthese, die sich entlang des Daumenbogens erstreckt und entlang dieser Erstreckung einen Querschnitt aufweist, der gewölbt ist, womit die Orthese mit den gewölbten Seitenwangen auch auf beiden Seiten des Daumenbogens an diesem anliegt. Anstelle der Fixierung gemäß den Druckschriften JP 2012-057286 A und JP 2005-074078 A schlägt JP 2017-080354 A vor, dass die dem Daumenbogen zugewandte Seite der Daumenorthese mit einem Klebstoff beschichtet ist und die Daumenorthese im Bereich des Daumenbogens an die Hand angeklebt ist.

JP 2011-194187 A schlägt eine T-förmige Orthese aus einem elastischen Kunststoffmaterial vor. Der Horizontalschenkel des T kann um ein Handgelenk geschlungen werden und die beiden Endbereiche des Horizontalschenkels des T können in so an die Hand angelegtem Zustand über ein Befestigungsmittel miteinander verbunden werden. Hingegen erstreckt sich von dem freien Endbereich des Vertikalschenkels des T eine schlaufenartige Aufnahmeösen für einen Finger der Hand. Für andere Ausführungsbeispiele erstrecken sich von dem Horizontalschenkel des T und parallel zu dem Vertikalschenkel des T (oder leicht gegenüber diesem geneigt) weitere Schenkel, die weitere Aufnahmeösen für andere Finger der Hand aufweisen.

### AUFGABE DER ERFINDUNG

Der vorliegenden Erfindung liegt die Aufgabe zugrunde, eine Daumenorthese vorzuschlagen, die hinsichtlich
- der Gewährleistung einer Grundstellung und/oder
- Möglichkeiten zur Veränderung der Lage des Daumens gegenüber der Grundstellung und/oder
- des Tragekomforts und/oder
- der Baugröße und/oder
- des optischen Erscheinungsbilds
verbessert ist. Des Weiteren liegt der Erfindung die Aufgabe zugrunde, ein Set mit mehreren Daumenorthesen, die wie zuvor erläutert verbessert sind, vorzuschlagen.

### LÖSUNG

Die Aufgabe der Erfindung wird erfindungsgemäß mit den Merkmalen der unabhängigen Patentansprüche gelöst. Weitere bevorzugte erfindungsgemäße Ausgestaltungen sind den abhängigen Patentansprüchen zu entnehmen.

### BESCHREIBUNG DER ERFINDUNG

Die Erfindung betrifft eine Daumenorthese, die zunächst einen Spreizkörper aufweist, der dem Auseinanderspreizen eines Daumens und eines Zeigefingers dient, also die Grundstellung gewährleistet soll. Des Weiteren weist die erfindungsgemäße Daumenorthese zwei Halteeinrichtungen auf. Mittels einer ersten Halteeinrichtung kann ein erster Endbereich des Spreizkörpers an dem Daumen gehalten werden. Hingegen kann mit einer zweiten Halteeinrichtung ein zweiter Endbereich des Spreizkörpers an dem Zeigefinger gehalten werden. Die Erfindung schlägt vor, dass die Daumenorthese ausschließlich über die beiden Halteeinrichtungen an der Hand der die Daumenorthese tragenden Person gehalten ist.

Vorzugsweise besteht die Daumenorthese ausschließlich aus dem Spreizkörper und den beiden Halteeinrichtungen, womit sich ein besonders einfacher Aufbau derselben ergibt.

Erfindungsgemäß ist ermöglicht, dass sich der Spreizkörper der Daumenorthese ausschließlich oder vorrangig in dem Zwischenraum zwischen dem Daumen und dem Zeigefinger und/oder im Bereich des Daumenbogens erstreckt. Erfindungsgemäß kann vermieden werden, dass die Daumenorthese als eine Art "Handschuh" ausgebildet sein muss oder sich ein Halteband von dem Spreizkörper um die gesamte Hand erstrecken muss. Vielmehr erstreckt sich die Daumenorthese ausschließlich seitlich des Daumens, seitlich des Zeigefinders und im Bereich des Daumenbogens.

Obwohl die erfindungsgemäße Daumenorthese mit dem Spreizkörper den gewünschten Effekt, nämlich das Auseinanderspreizen des Daumens und des Zeigefingers in eine angestrebte Grundstellung, gewährleisten kann, ergibt sich eine sehr kleine Baugröße der Daumenorthese, die damit auch leicht gestaltet werden kann.

Des Weiteren ist der von der Daumenorthese beeinträchtigte oder abgedeckte Bereich der Hand gegenüber den von Daumenorthesen gemäß dem Stand der Technik abgedeckten Bereichen reduziert, womit auch Beeinträchtigungen der Hand reduziert sein können und der Bereich der Hand, in welchem die Daumenorthese sichtbar ist, auf das notwendige Minimum reduziert ist.

Unter Umständen kann infolge der klein bauenden Orthese auch eine Reduzierung des Materialeinsatzes erfolgen, womit die Kosten der Orthese reduziert werden können.

Erfindungsgemäß ist der Spreizkörper gezielt mit einer Elastizität ausgestattet. Hierbei wird die Elastizität des Spreizkörpers derart bemessen, dass es möglich ist, den Spreizkörper so elastisch zu verformen, dass der Abstand der Endbereiche des Spreizkörpers verringert werden kann. Hierbei erfolgt diese Verformung durch von dem Daumen und dem Zeigefinger aufgebrachte Schließkräfte. Die so herbeigeführte Verringerung des Abstands der Endbereiche des Spreizkörpers haben zur Folge, dass sich auch der an den Endbereichen des Spreizkörpers über die erste Halteeinrichtung angebundene Daumen in Richtung des Zeigefingers bewegen kann, womit trotz Einsatzes der Daumenorthese ein Greifen eines Gegenstands zwischen Daumen und Zeigefinger und anderweitige Bewegungen des Daumens möglich sind, wobei insbesondere auch der sogenannte Pinzettengriff infolge der Elastizität ermöglicht, vereinfacht oder verbessert werden kann.

Grundsätzlich kann der Spreizkörper eine beliebige Geometrie und insbesondere einen beliebigen Verlauf der Längsachse und/oder des Querschnitts aufweisen. Für einen Vorschlag der Erfindung weist der Spreizkörper (in einer Draufsicht) (und in einer u. U. schematischen Betrachtungsweise) einen gekrümmten Grundschenkel und zwei Seitenschenkel auf. Die Seitenschenkel erstrecken sich jeweils von einem zugeordneten Endbereich des Grundschenkels und divergieren mit zunehmendem Abstand von dem Grundschenkel. Hierbei ist der gekrümmte Grundschenkel des Spreizkörpers dazu bestimmt, an dem Daumenbogen zur Anlage zu kommen. Hingegen kommt an einem Seitenschenkel der Daumen zur Anlage, während an dem anderen Seitenschenkel der Zeigefinger zur Anlage kommt. In den Endbereichen der Seitenschenkel erfolgt dann die Verbindung des Spreizkörpers über die Halteeinrichtungen mit dem Daumen bzw. Zeigefinger. Die Elastizität des Spreizkörpers ist auch oder nur im Bereich des gekrümmten Grundschenkels vorhanden, womit es möglich ist, durch von dem Daumen und dem Zeigefinger aufgebrachte Kräfte den Grundschenkel zu verformen, womit der Öffnungswinkel, mit dem in der unbelasteten Grundstellung der Daumen von dem Zeigefinger gespreizt wird, verändert werden kann.

Erfindungsgemäß kann sich der Spreizkörper kaum sichtbar und unter guter Führung sowie großflächiger Anlage von dem Daumen über den Daumenbogen zu dem Zeigefinger erstrecken, was den Tragekomfort verbessert und/oder dazu führt, dass die Daumenorthese wenig sichtbar ist und kein Hindernis bei der Benutzung der Hand darstellt.

Je nach gewünschter Grundstellung von Daumen und Zeigefinger können die Seitenschenkel einen beliebigen Öffnungswinkel aufweisen. Vorzugsweise beträgt der Öffnungswinkel der Seitenschenkel 60°, wobei der Öffnungswinkel aber auch um ± 20 % (insbesondere ± 10 % oder ± 5 %) von einem Winkel von 60° abweichen kann. Es hat sich herausgestellt, dass eine hierdurch gewährleistete Öffnungsstellung von Zeigefinger und Daumen eine besonders gute therapeutische Wirkung bereitstellt, wobei unter Umständen dennoch ein sogenannter "Pinzettengriff" möglich bleibt.

Der Spreizkörper kann einen beliebigen (über die Erstreckung konstanten oder veränderlichen) Querschnitt aufweisen. Für einen besonderen Vorschlag kann der Spreizkörper einen gekrümmten Querschnitt aufweisen. Hierbei ist der gekrümmte Querschnitt insbesondere in dem Kontaktbereich mit dem Daumen, dem Daumenbogen und/oder dem Zeigefinger auf der dem Daumen, dem Daumenbogen und/oder Zeigefinger zugewandten Seite konkav ausgebildet, so dass der Querschnitt den Daumen, den Daumenbogen und/oder den Zeigefinger teilweise umschließen oder aufnehmen kann. Die Umschließung führt einerseits zu einer Vergrößerung der Kontaktfläche, womit Beeinträchtigungen der Haut und Druckspitzen vermieden werden können. Andererseits führt die infolge der teilweisen Umschließung formschlüssige Aufnahme des Daumens, des Daumenbogens und/oder des Zeigefingers in dem konkav gekrümmten Querschnitt des Spreizkörpers zu einer guten Führung und/oder Fixierung der Daumenorthese gegenüber dem Daumen, dem Daumenbogen und/oder dem Zeigefinger.

Für die Herstellung des Spreizkörpers kann bspw. ein beliebiges Material, ein Materialgemisch oder ein Verbundmaterial Einsatz finden. Für einen Vorschlag der Erfindung ist der Spreizkörper aus Kunststoff hergestellt, was beispielsweise in einem Spritzgussverfahren erfolgen kann.

Für die Ausgestaltung der Halteeinrichtungen, über welche der Spreizkörper an dem Daumen bzw. dem Zeigefinger befestigt wird, gibt es vielfältige Möglichkeiten, wobei die aus dem Stand der Technik bekannten Halteeinrichtungen eingesetzt werden können. Für einen Vorschlag der Erfindung weist mindestens eine Halteeinrichtung einen (beispielsweise flexiblen, elastischen und/oder formstabilen) Haltestreifen auf. Der Haltestreifen umschließt den zugeordneten Daumen oder Zeigefinger zumindest teilweise, wobei der Haltestreifen auch ringförmig den Daumen oder den Zeigefinger vollständig umschließen kann. Möglich ist auch, dass die Halteeinrichtung ausgehend von dem Spreizkörper zwei Haltestreifen oder einen Haltestreifen mit zwei Haltestreifenabschnitten aufweist, die sich in entgegengesetzte Umfangsrichtungen von dem Spreizkörper um den Daumen oder den Zeigefinger erstrecken.

Für die Ausgestaltung der Haltestreifen gibt es im Rahmen der Erfindung vielfältige Möglichkeiten. Um ledig einige Möglichkeiten zu nennen, kann der Haltestreifen ein elastisch in Längsrichtung verformbarer Haltestreifen und/oder ein elastisch biegbarer Haltestreifen sein, beispielsweise ein Haltestreifen aus Gummi. Mit der Elastizität kann dann ein Verspannen der Halteeinrichtung mit dem Daumen oder dem Zeigefinger gewährleistet werden, womit der Halt der Daumenorthese an der Hand verbessert werden kann. Möglich ist auch, dass ein (unter Umständen auch mit einer Erwärmung) plastisch verformbarer Haltestreifen Einsatz findet, wobei durch die plastische Verformung eine Anpassung der grundsätzlich formstabilen Haltestreifen an die Größe und/oder Geometrie der Daumens oder des Zeigefingers unterschiedlicher Personen erfolgen kann. Natürlich ist auch möglich, dass ein Haltestreifen Einsatz findet, der für eine Anpassbarkeit an die unterschiedlichen Benutzer plastisch verformbar ist und dann dennoch eine gewisse Elastizität bereitstellt, mit der eine gute und unter Umständen enge oder straffe Halteverbindung zwischen dem Daumen oder dem Zeigefinger und dem Haltestreifen gewährleistet werden kann. Möglich ist auch, dass ein textiler Haltestreifen Einsatz findet oder ein Klettband.

In weiterer Ausgestaltung der erfindungsgemäßen Daumenorthese sind Endbereiche von einem Haltestreifen der Halteeinrichtung oder von zwei Haltestreifen der Halteeinrichtung über eine Verbindungseinrichtung miteinander verbindbar. Hierbei gewährleistet die Verbindungseinrichtung vorzugsweise eine Verbindung der beiden Endbereiche in unterschiedlichen Relativstellungen, wodurch ermöglicht werden kann, dass die Halteeinrichtung an den Daumen oder den Zeigefinger angelegt wird und auch je nach Relativstellung eine Anpassung an unterschiedliche Abmessungen eines Daumens oder eines Zeigefingers erfolgen kann oder auch unterschiedliche Spannungen gegenüber dem Daumen oder dem Zeigefinger herbeigeführt werden können. Bilden die Haltestreifen einen Ring, kann über die unterschiedlichen Relativstellungen der Verbindungseinrichtung, in denen die Verbindung der beiden Endbereiche erfolgt, der Durchmesser des bereitgestellten Rings eingestellt werden. Um lediglich einige, die Erfindung nicht beschränkende Beispiele zu nennen, kann die Verbindungseinrichtung als Klettverschluss ausgebildet sein. Möglich ist aber auch, dass die Verbindungseinrichtung ein von einem Endbereich eines Haltestreifens oder des Spreizkörpers ausgebildetes Steckelement, beispielsweise einen Pilzkopf aufweist. Des Weiteren verfügt die Verbindungseinrichtung über eine von dem Haltestreifens (insbesondere in einem Endbereich) ausgebildete Ausnehmung. Das Steckelement wird zur Herstellung der Verbindung in die Ausnehmung eingesteckt. Für das genannte Beispiel des Pilzkopfs handelt es sich bei der Ausnehmung um eine Bohrung oder ein Loch des Haltestreifens, in welches der insbesondere von dem Spreizkörper getragene Pilzkopf eingesteckt werden kann. Möglich ist auch, dass Ausnehmungen in beiden Endbereichen des Haltestreifens mit dem Pilzkopf verbunden werden. Sollen unterschiedliche Relativstellungen möglich sein, können über die Längserstreckung der Haltestreifen mehrere Steckelemente und/oder Ausnehmungen angeordnet sein.

Für die Art der Kopplung der Halteeinrichtungen mit dem Spreizkörper gibt es beliebige Möglichkeiten. Im einfachsten Fall ist der Spreizkörper fest mit der Halteeinrichtung verbunden, beispielsweise durch einen Stoffschluss, vernäht, verklebt, verschraubt u. ä. Die Erfindung schlägt aber auch vor, dass die Verbindung der Halteeinrichtung mit dem Spreizkörper über eine Kopplungseinrichtung erfolgt. Diese Kopplungseinrichtung kann beispielsweise lösbar sein, so dass unter Umständen möglich ist, dass dieselben Halteeinrichtungen mit unterschiedlichen Spreizkörpern (beispielsweise Spreizkörpern unterschiedlicher Abmessungen und/oder unterschiedlicher Steifigkeiten) eingesetzt werden können. Unter Umständen kann eine lösbare Kopplungseinrichtung auch ein verändertes Anlegen der Orthese ermöglichen, indem zunächst eine Verbindung der Halteeinrichtungen mit dem Daumen und dem Zeigefinger erfolgt und dann erst über die Kopplungseinrichtungen der Spreizkörper mit den Halteeinrichtungen verbunden wird. Alternativ oder zusätzlich möglich ist, dass die Kopplungseinrichtung einen Freiheitsgrad bereitstellt. Möglich ist beispielsweise, dass infolge der elastischer Ausgestaltung des Spreizkörpers die elastische Verformung desselben zur Folge hat, dass sich eine Relativbewegung zwischen der Hand und dem Spreizkörper ergibt. Ist in diesem Fall die Halteeinrichtung ohne Freiheitsgrad der Kopplungseinrichtung mit dem Spreizkörper verbunden, kann dies zu einem Scheuern der Halteeinrichtung an dem Daumen oder dem Zeigefinger führen. Zur Vermeidung eines derartigen Scheuerns kann ein Verschiebe-Freiheitsgrad der Kopplungseinrichtung eine Ausgleichsbewegung gewährleisten, so dass sich trotz elastischer Verformung des Spreizkörpers der Kontaktbereich der Halteeinrichtung mit dem Daumen und dem Zeigefinger nicht ändert und auch keine Reibkräfte in dem Kontaktbereich erzeugt werden.

Für einen besonderen Vorschlag verfügt der Spreizkörper (insbesondere auf der dem Daumen, dem Daumenbogen oder dem Zeigefinger abgewandten Seite) über ein Führungselement. Mittels des Führungselements kann eine Führung eines Gegenstands erfolgen, der mit der Hand, die mit der Daumenorthese ausgestattet ist, getragen oder gegriffen wird. Bei dem Führungselement kann es sich beispielsweise um eine Führungsrippe, einen Führungsvorsprung oder eine Führungsvertiefung handeln. Soll beispielsweise mit der die Daumenorthese tragenden Hand geschrieben werden, wird üblicherweise der genutzte Stift an den Daumenbogen der Hand angelegt, wobei über die Anpresskraft des Daumenbogens an den Stift eine Fixierung und Vorgabe der Ausrichtung des Stifts erfolgen kann. Liegt hingegen der Stift an dem Spreizkörper an, könnte hier die Fixierung und Vorgabe der Orientierung reduziert sein. In diesem Fall kann mit einem Führungselement wie einer Führungsrippe eine formschlüssige Abstützung des Stifts erfolgen, womit das Schreiben mit dem Stift erleichtert werden kann.

Grundsätzlich können beliebige Formgebungen und Dimensionierungen der erfindungsgemäßen Daumenorthese und insbesondere des Spreizkörpers erfolgen, wobei vorzugsweise eine Anpassung der Dimensionierung der Daumenorthese an die Größe der Hand des Benutzers erfolgt. Möglich ist auch, dass Daumenorthesen in einer begrenzten Anzahl von unterschiedlichen Abmessungen bereitgestellt werden, welche dann für zugeordnete Größen der Hände in spezifizierten Abmessungsbereich bestimmt sind.

Für einen Vorschlag der Erfindung ist die Daumenorthese wie folgt dimensioniert:
a) Für einen Vorschlag der Erfindung weist der Spreizkörper einen gekrümmten Grundschenkel auf, dessen Krümmungsradius 25 mm beträgt. Da der gekrümmte Grundschenkel für die Anlage an den Übergang oder Daumenbogen bestimmt ist, entspricht der Krümmungsradius von 25 mm dem Krümmungsradius des Daumenbogens.
b) Alternativ oder zusätzlich weist der Spreizkörper einen ersten Seitenschenkel auf, der für die Anlage an den Daumen bestimmt ist. Die Länge dieses ersten Seitenschenkels beträgt 11 mm.
c) Alternativ oder zusätzlich weist der Spreizkörper einen zweiten Seitenschenkel auf, dessen Länge 30 mm beträgt. Dieser zweite Seitenschenkel ist, wie oben erläutert, für die Anlage an den Zeigefinger bestimmt.
d) Alternativ oder zusätzlich weist der Spreizkörper im Bereich des ersten Seitenschenkels einen Querschnitt mit einem Krümmungsradius von 18 mm auf. Dieser Krümmungsradius entspricht ungefähr dem Durchmesser eines Daumens in dem Anlagebereich des ersten Seitenschenkels.
e) Alternativ oder zusätzlich kann der Spreizkörper im Bereich des zweiten Seitenschenkels einen Querschnitt mit einem Krümmungsradius von 37 mm aufweisen, was ungefähr dem Krümmungsradius des Zeigefingers entspricht.
f) Alternativ oder zusätzlich kann der Spreizkörper im Bereich des Grundschenkels, der für die Anlage an den Daumenbogen bestimmt ist, einen Krümmungsradius von 12 mm aufweisen.

Die vorgenannten Abmessungen sind vorzugsweise für eine Hand einsetzbar, bei der der Daumenbogen für abgespreizten Zeigefinder und Daumen einen Radius von 25 mm +/- 3 mm oder 25 mm +/- 2 mm oder 25 mm +/- 1 mm aufweist, wobei mit entsprechender Skalierung für eine andere Handgröße entsprechend angepasste Abmessungen zum Einsatz kommen können. Im Rahmen der Erfindung sind auch Abweichungen der angegebenen Längen und/oder Radien von ±50 %, ± 20 %, ± 10 % oder ± 5 % möglich. Hierbei können die zuvor spezifizierten Abmessungen a) bis f) sämtlich oder nur teilweise an einer Daumenorthese verwirklicht sein.

Wie zuvor erwähnt, können Daumenorthesen unterschiedlicher Abmessungen für unterschiedliche Hände bereitgestellt werden. Für einen Vorschlag der Erfindung kann aber auch eine Anpassung einer existierenden Daumenorthese dadurch erfolgen, dass die Länge mindestens eines Seitenschenkels des Spreizkörpers und/oder die Größe einer Halteeinrichtung oder die Länge mindestens eines Haltestreifens veränderbar ist. Möglich ist dabei, dass eine Veränderung der Länge durch eine Solltrennstelle, insbesondere eine Materialschwächung, des Seitenschenkels des Spreizkörpers oder des Haltestreifens ermöglicht ist. Stellt sich für eine Hand, die mit der Daumenorthese ausgestattet werden soll, heraus, dass der Seitenschenkel des Spreizkörpers zu lang ist, kann der Seitenschenkel durch Durchtrennen oder Durchbrechen der Solltrennstelle gekürzt werden. Das Entsprechende gilt für den Haltestreifen. Durch dieses Abtrennen überschüssiger Materialbereiche kann das optische Erscheinungsbild der Daumenorthese verbessert werden, das Gewicht der Daumenorthese wird auf das erforderliche Ausmaß reduziert und es können auch Beeinträchtigungen durch die überstehenden Materialbereiche vermieden werden.

Eine weitere Lösung der der Erfindung zugrunde liegenden Aufgabe stellt ein Set dar, welches mehrere unterschiedliche Daumenorthesen aufweist. Ein derartiges Set unterschiedlicher Daumenorthesen kann beispielsweise von einem Hersteller bereitgestellt werden oder auch von einem Orthopädiefachhandel vorgehalten werden, so dass für Kunden mit unterschiedlicher Größe der Hände jeweils eine passende Daumenorthese bereitgestellt ist. In dem Set mit den mehreren Daumenorthesen weisen die unterschiedlichen Daumenorthesen unterschiedliche Abmessungen für Hände unterschiedlicher Größen auf.

Alternativ oder zusätzlich möglich ist, dass die unterschiedlichen Daumenorthesen des Sets Spreizkörper aufweisen, die unterschiedliche Elastizitäten aufweisen. Hierdurch ist ermöglicht, dass eine Daumenorthese mit einem Spreizkörper mit einer sehr geringen Elastizität gewählt wird, wenn eine Fixierung des Daumens gewünscht ist, während je nach Ausmaß der gewünschten Flexibilität des Spreizkörpers dann Spreizkörper mit einer größeren Elastizität Einsatz finden können. Möglich ist auch, dass über die Wahl der Elastizität des Spreizkörpers den jeweils von der Hand erzeugbaren Handkräften unterschiedlicher Patienten Rechnung getragen wird.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Element die Rede ist, ist dies so zu verstehen, dass genau ein Element, zwei Elemente oder mehr Elemente vorhanden sind. Diese Merkmale können durch andere Merkmale ergänzt werden oder die einzigen Merkmale sein, aus denen das jeweilige Erzeugnis besteht.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1 bis 7**: zeigen unterschiedliche Ansichten eines Spreizkörpers einer Daumenorthese, wobei den Fig. 4 bis 7 mögliche Abmessungen des Spreizkörpers entnommen werden können.
- **Fig. 8 bis 11**: zeigen eine Daumenorthese mit einem Spreizkörper gemäß Fig. 1 bis 7 und Halteeinrichtungen in einem an die Hand angelegten Zustand in unterschiedlichen Betriebsstellungen.
- **Fig. 12 bis 14**: zeigen Daumenorthesen mit unterschiedlichen Ausgestaltungen der Halteeinrichtungen in angelegtem Zustand.

- **Fig. 15 und 16**: zeigen eine weitere Ausgestaltung einer Daumenorthese mit einem sich nicht entlang des Daumenbogens, sondern zwischen Daumen und Zeigefinger erstreckenden Spreizkörper.
- **Fig. 17**: zeigt stark schematisch eine Ermittlung einer Steifigkeit einer Daumenorthese.

### FIGURENBESCHREIBUNG

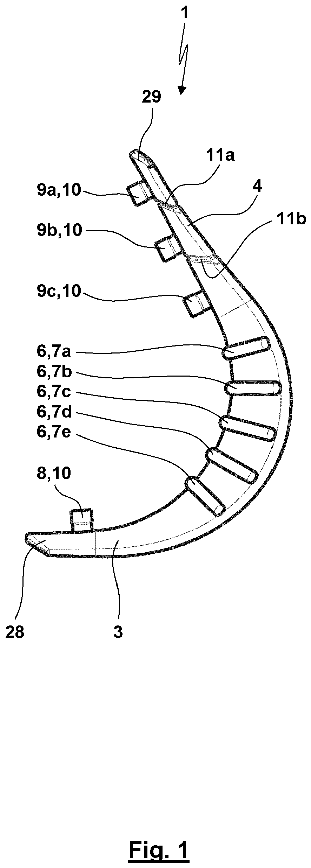

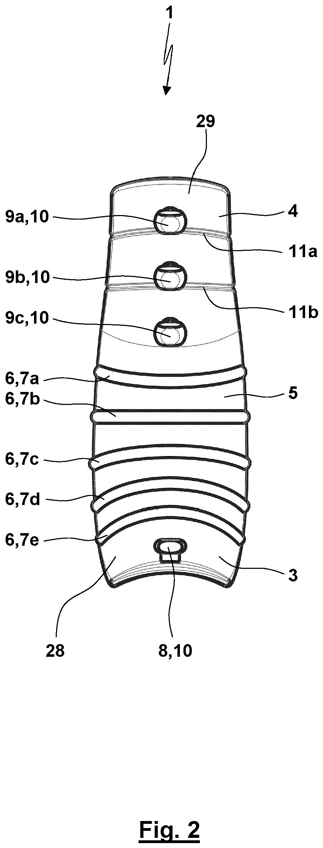

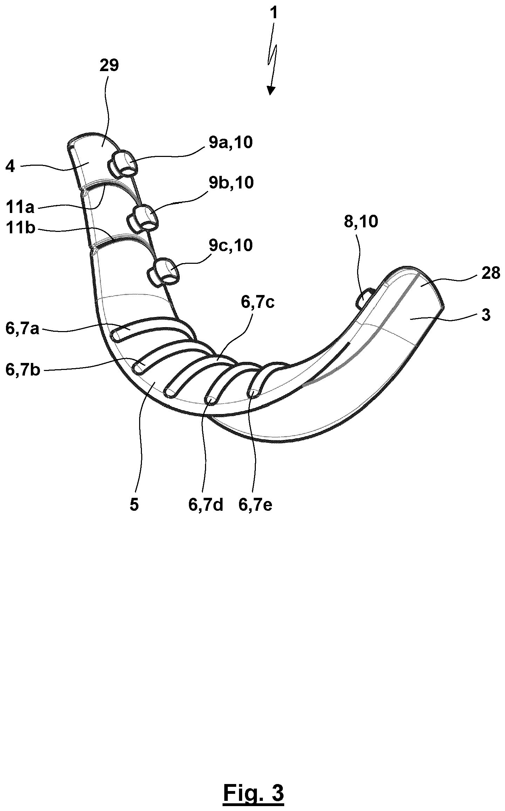

In den **Fig. 1 bis 7** ist ein Spreizkörper 1 einer Daumenorthese 2 dargestellt. Der Spreizkörper 1 verfügt (in der Draufsicht gemäß Fig. 1) über zwei grundsätzlich geradlinige Seitenschenkel 3, 4, die über einen gekrümmten Grundschenkel 5 miteinander verbunden sind. Der Spreizkörper 1 ist in seinem Querschnitt derart gewölbt, dass der Spreizkörper 1 im Kontaktbereich mit der Hand konkav ist.

Hierbei ändert sich die konkave Krümmung des Querschnitts über die Längserstreckung von dem Seitenschenkel 3 über den Grundschenkel 5 zu dem Seitenschenkel 4. Die Quererstreckung entlang der Wölbung ändert sich ebenfalls über die Längserstreckung des Spreizkörpers 1, wobei die Quererstreckung des Spreizkörpers 1 im Bereich des gekrümmten Grundschenkels 5 größer ist als im Bereich der Seitenschenkel 3, 4. Vorzugsweise nimmt die Quererstreckung des Spreizkörpers 1 von der maximalen Breite ungefähr in der Mitte des Grundschenkels 5 kontinuierlich ab bis zu den freien Endbereichen der Seitenschenkel 3, 4. Optional kann der Spreizkörper 1 auf der Seite, welche nicht die Kontaktfläche mit der Hand ausbildet, mit Führungselementen 6, hier Führungsrippen 7a, 7b, 7c, 7d, 7e, ausgestattet sein.

Optional verfügt der Spreizkörper 1 im Bereich des Seitenschenkels 3 und/oder im Bereich des Seitenschenkels 4 über mindestens eine Kopplungseinrichtung 8 bzw. 9a, 9b, 9c. Für das dargestellte Ausführungsbeispiel sind die Kopplungseinrichtungen 8, 9 integral von dem Spreizkörper 1 ausgebildete Pilzköpfe 10.

Möglich ist, dass im Bereich mindestens eines Seitenschenkels 3, 4 Solltrennstellen 11a, 11b vorgesehen sind. Die Solltrennstellen 11a, 11b ermöglichen eine Verringerung der Länge des zugeordneten Seitenschenkels 3, 4. Möglich ist, dass mit einer Verkürzung der Länge eines Seitenschenkels 3, 4 auch eine Abtrennung einer Kopplungseinrichtung 9a, 9b erfolgt. Für das dargestellte Ausführungsbeispiel sind die Solltrennstellen 11a, 11b als Materialaussparungen oder-schwächungen in Form von Nuten ausgebildet.

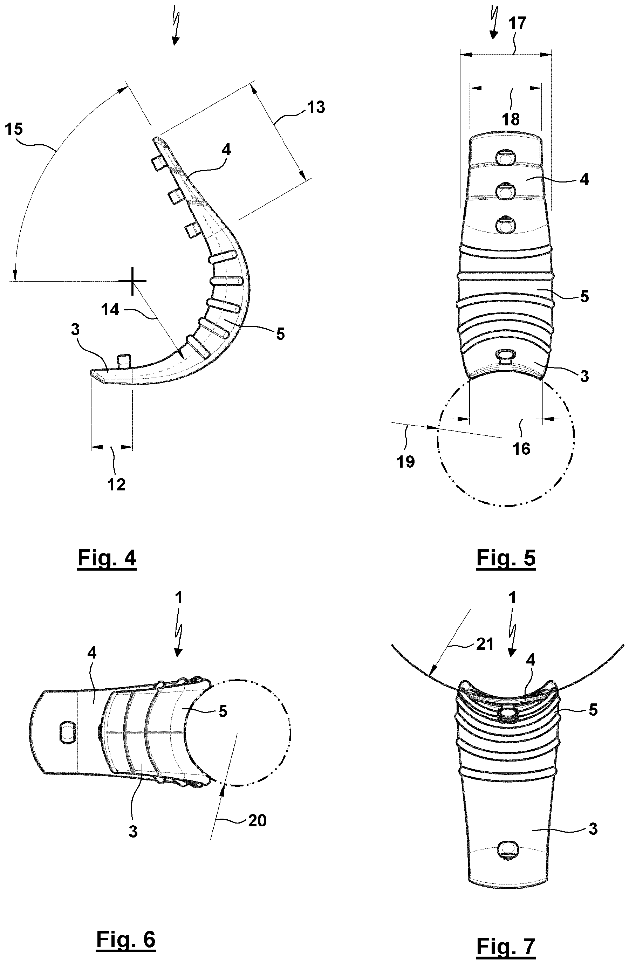

Aus den Fig. 4 bis 7 sind mögliche Dimensionen des Spreizkörpers 1 zu dargestellt. So beträgt eine Länge 12 des Seitenschenkels 3 11 mm, während eine Länge 13 des Seitenschenkels 4 30 mm betragen kann. Der Krümmungsradius 14 des Grundschenkels 5 kann 25 mm betragen. Ein Öffnungswinkel 15 der beiden Seitenschenkel 3, 4 kann 60° betragen, was durch den entsprechenden Umfangswinkel und die Krümmung des Grundschenkels 5 gewährleistet wird. Die Quererstreckung des Spreizkörpers 1 verändert sich über dessen Längserstreckung. Die kleinste Quererstreckung 16 des Seitenschenkels 3 in dessen freiem Endbereich kann 19 mm betragen, während die größte Quererstreckung 17 des Grundschenkels 5 24 mm betragen kann und die kleinste Quererstreckung 18 des Seitenschenkels 4 in dessen freiem Endbereich 19 mm betragen kann. Zwischen diesen minimalen und maximalen Quererstreckungen 16, 17, 18 ändert sich die Quererstreckung kontinuierlich. Im Bereich des Seitenschenkels 3 ist der Querschnitt des Spreizkörpers 1 mit einem Krümmungsradius 19 gekrümmt, der 19 mm betragen kann. Im Bereich des Grundschenkels 5 kann der Krümmungsradius 20 12 mm betragen. Im Bereich des Seitenschenkels 4 kann der Krümmungsradius 21 37 mm betragen.

Je nach Größe der Hand, des Daumens, des Zeigefingers und/oder der Daumenbeuge können die Abmessungen angepasst werden, wobei die vorgenannten Abmessungen vorzugsweise für eine Handgröße 25 gelten. Möglich ist, dass der Öffnungswinkel 15 um ± 20° (vorzugsweise ± 15° oder ± 10° oder ± 5°) von dem angegebenen Öffnungswinkel von 60° abweicht. Hinsichtlich der anderen Abmessungen gilt, dass diese um ± 50 %, ± 20 %, ± 15 %, ± 10 % oder ± 5 % von den angegebenen Abmessungen abweichen können.

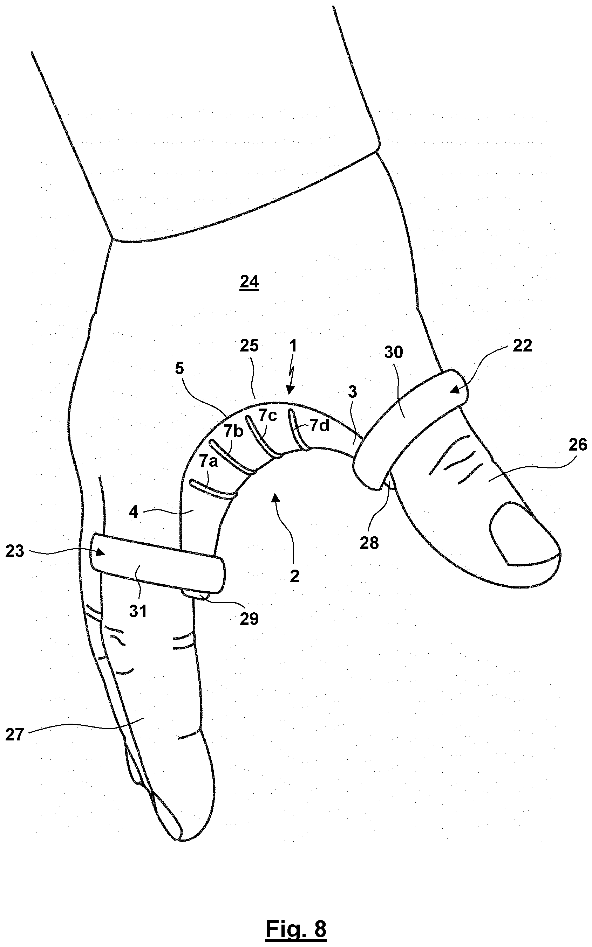

**Fig. 8 bis 11** zeigen eine Daumenorthese 2, die aus dem Spreizkörper 1 gemäß Fig. 1 bis 7 sowie endseitig daran angebrachten Halteeinrichtungen 22, 23 besteht und an eine Hand 24 angelegt ist. Hierbei liegt der Spreizkörper 1 mit der konkav gewölbten Innenseite des Grundschenkels 5 an dem Übergang von dem Daumen 26 zu dem Zeigefinger 27, der hier als Daumenbogen 25 bezeichnet wird, an. Infolge der konkaven Wölbung der dem Daumenbogen 25 zugewandten Seite des Spreizkörpers 1 umschließt der Querschnitt des Grundschenkels 5 teilweise den Daumenbogens 25. Anders gesagt tritt der Daumenbogen 25 in die konkave Vertiefung des Grundschenkels 5 ein.

An dem Seitenschenkel 3 liegt der Daumen 26 an. Vorzugsweise ist die Länge des Seitenschenkels 3 derart bemessen, dass sich dieser ungefähr bis zu dem vordersten Daumengelenk erstreckt oder kurz hiervor endet. Infolge der konkaven Wölbung des Seitenschenkels 3 umschließt dieser auch teilweise im Querschnitt den Daumen 26.

An dem Seitenschenkel 4 liegt der Zeigefinger 27 an. Infolge der konkaven Wölbung der dem Zeigefinger 27 zugewandten Seite des 4 umschließt der Querschnitt des Seitenschenkels 4 teilweise den Zeigefinger 27. Die Länge des Seitenschenkels 4 ist vorzugsweise derart bemessen, dass sich dieser ungefähr bis zu dem zweit-vordersten Gelenk des Zeigefingers 27 erstreckt oder kurz hiervor endet.

In einem freien Endbereich 28 trägt der Seitenschenkel 3 die Halteeinrichtung 22. Entsprechend trägt ein freier Endbereich 29 des Seitenschenkels 4 die Halteeinrichtung 23.

Die Halteeinrichtungen 22, 23 verfügen jeweils über einen Haltestreifen 30, 31. Der Haltestreifen 30 ist ring- oder schlaufenartig ausgebildet und umgibt vollständig den Daumen 26. Unter insbesondere elastischer Vorspannung wird von dem Haltestreifen 30 der Seitenschenkel 3 an den Daumen 26 gepresst. Das Entsprechende gilt hinsichtlich des Haltestreifens 31 und des Zeigefingers 27.

Fig. 8 zeigt die Hand 24 mit der Daumenorthese 2 in angelegtem Zustand, wobei der Benutzer keine Kraft auf den Daumen 26 und den Zeigefinger 27 aufbringt, so dass die Ausrichtung des Daumens 26 und des Zeigefingers 27 durch den Spreizkörper 1 vorgegeben ist entsprechend dem Öffnungswinkel 15, womit eine Grundstellung gewährleistet ist.

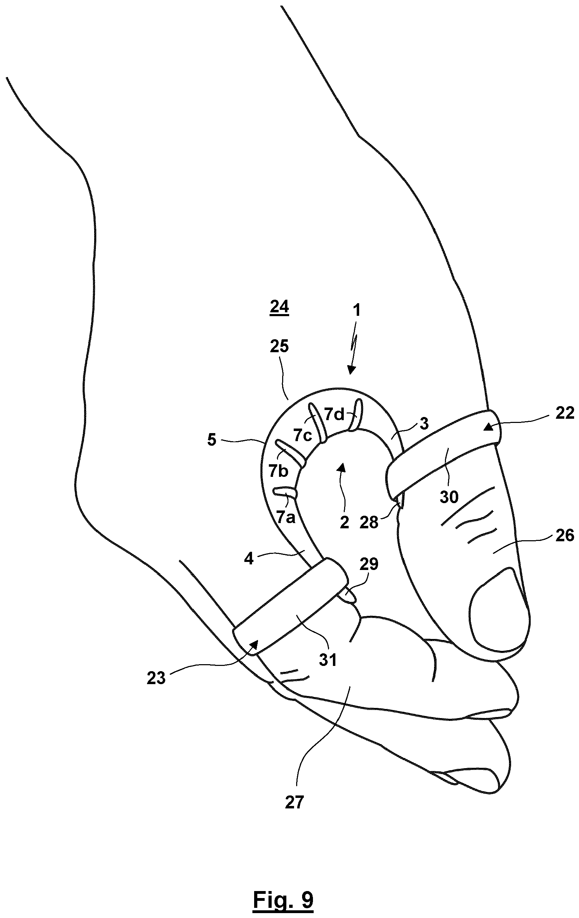

Fig. 9 zeigt die Daumenorthese 2 in einem Zustand, in welchem der Spreizkörper 1 elastisch verformt ist, indem der Benutzer Kräfte erzeugt, die den Daumen 26 in Richtung des Zeigefingers 27 bewegen. Die erzeugten Kräfte führen zur elastischen Verformung des Spreizkörpers insbesondere im Bereich des Grundschenkels 5, womit sich der Öffnungswinkel 15 verringert und damit der Abstand des Daumens 26 von dem Zeigefinger 27 (im Bereich von deren Anlagefläche an den Seitenschenkeln 3, 4) verringert werden kann. Fig. 9 zeigt den sogenannten "Pinzettengriff". Möglich ist dabei, dass sich der Öffnungswinkel von 60° in der Grundstellung infolge der aufgebrachten Kräfte bis auf 10°, 0° oder sogar gemäß Fig. 9 bis zu einem negativen Winkel derart, dass die Seitenschenkel 3, 4 nicht mehr divergieren, sondern konvergieren, ändert.

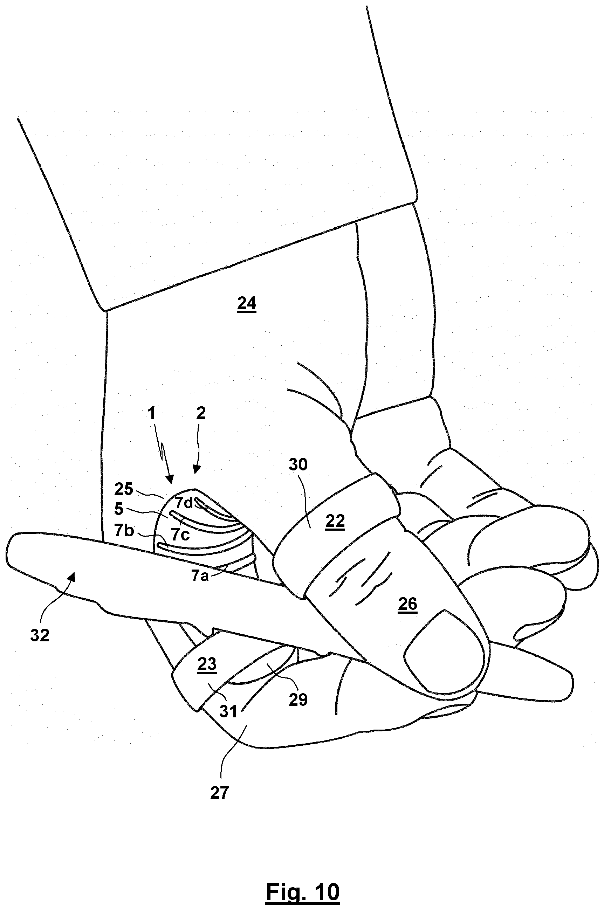

Fig. 10 zeigt die Benutzung der Daumenorthese 2 für das Schreiben mit einem Stift 32. Zu erkennen ist, dass der Stift 32 an den Führungsrippen 7 anliegt und durch diese formschlüssig geführt ist.

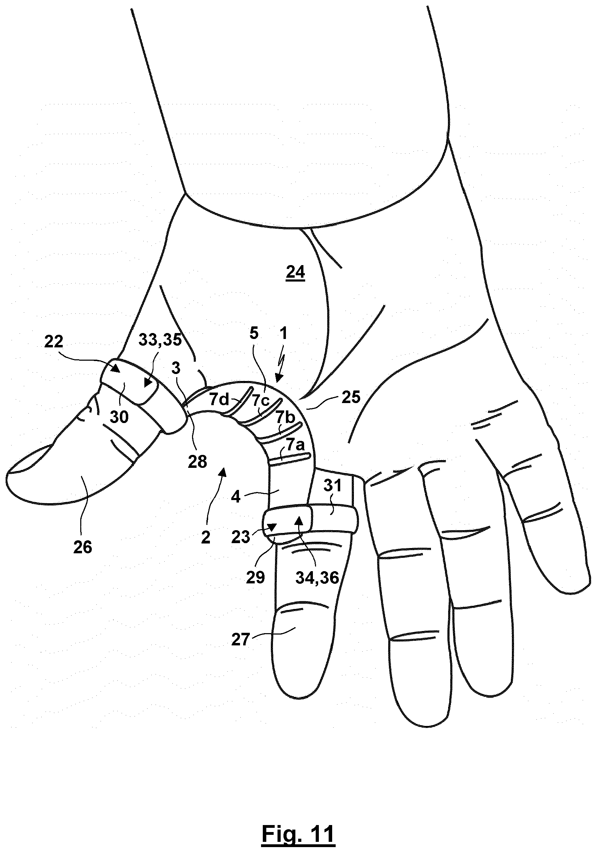

Für die in Fig. 11 dargestellte Ausführungsform sind die Haltestreifen 30, 31 endseitig mittels Verbindungseinrichtungen 33, 34 miteinander verbunden, die hier als Klettverschlüsse 35, 36 ausgebildet sind. Über die Klettverschlüsse 35, 36 können die Haltestreifen 30, 31 stramm um den Daumen 26 bzw. den Zeigefinger 27 geschlossen werden. In diesem Fall sind die Haltestreifen 30, 31 als textile Streifen oder Klettverschluss-Streifen ausgebildet, die fest mit dem Spreizkörper 1 verbunden sind, beispielsweise durch Verkleben oder Vernähen.

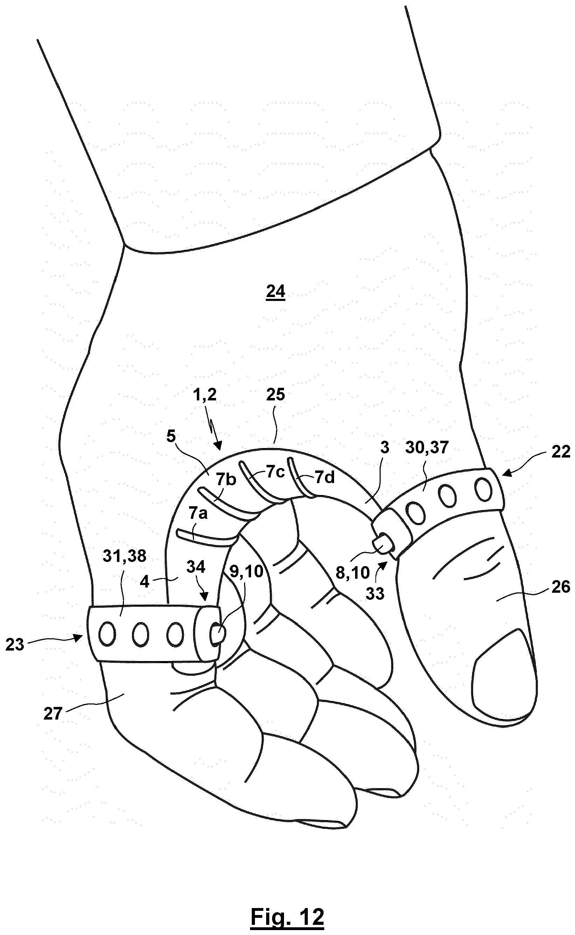

Für das in **Fig. 12** dargestellte Ausführungsbeispiel sind die Haltestreifen 30, 31 als Lochstreifen 37, 38 ausgebildet. Vorzugsweise sind in diesem Fall die Haltestreifen 30 elastisch, beispielsweise als Gummistreifen, ausgebildet. Ein Loch in einem Endbereich des Haltestreifens 30, 31 ist dann auf einem Pilzkopf 10 des Spreizkörpers 1 aufgesteckt und nach der Umschlingung des Daumens 26 bzw. des Zeigefingers 27 ist ein anderer Endbereich des Haltestreifens im Bereich eines weiteren Lochs auf den Pilzkopf 10 aufgesteckt. Da die Haltestreifen 30, 31 über eine Vielzahl von über die Längserstreckung verteilte Löcher verfügen, kann je nach Wahl des Lochs für das Aufstecken auf den Pilzkopf 10 eine Anpassung an unterschiedliche Durchmesser des Daumens 26 bzw. des Zeigefingers 27 und/oder an unterschiedliche gewünschten Anpresskräfte erfolgen. Möglich ist, dass die Haltestreifen 30, 31 auch über Solltrennstellen verfügen, über welche dann eine überschüssige Länge der Haltestreifen 30, 31 abgetrennt werden kann.

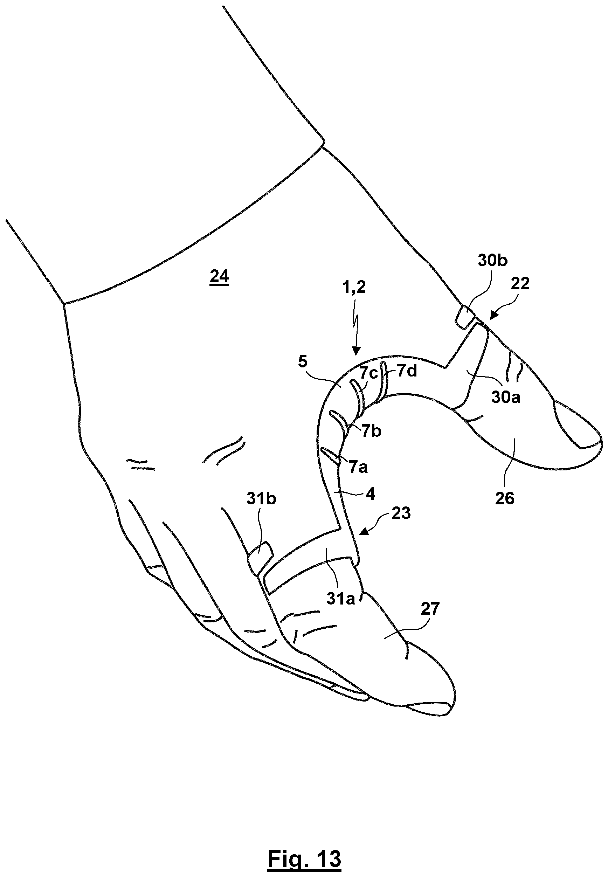

**Fig. 13** zeigt eine abweichende Ausgestaltung der Halteeinrichtungen 22, 23. Hier sind die Haltestreifen 30, 31 nicht flexible, sondern eigensteif ausgebildet. In diesem Fall erstrecken sich von dem Grundkörper des Spreizkörpers 1 in unterschiedliche Umfangsrichtung um den Daumen 26 bzw. den Zeigefinger 27 jeweils Haltestreifen30a, 30b bzw. 31a, 31b, wobei diese für das dargestellte Ausführungsbeispiel aneinander vorbeigeführt sind und somit eine Überlappung aufweisen, ohne dass dies zwingend der Fall ist. Die Haltestreifen 30a, 30b bzw. 31a, 31b verfügen über eine Elastizität und die eigensteife Form derselben ist derart vorgegeben, dass bei einem Einfädeln des Daumens 26 bzw. des Zeigefingers 27 die Haltestreifen 30a, 30b bzw. 31a, 31b elastisch aufgeweitet werden, womit die Haltestreifen 30a, 30b bzw. 31a, 31b in der angelegten Stellung mit dem Daumen 26 bzw. dem Zeigefinger 27 verspannt sind und so die Daumenorthese 2 an der Hand 24 fixieren. Möglich ist dabei, dass (wie in Fig. 13 dargestellt) die Haltestreifen 30a, 30b, 31a, 31b einstückig mit dem Spreizkörper 1 verbunden sind, was beispielsweise dann möglich ist, wenn der Spreizkörper 1 mit den Haltestreifen 30a, 30b, 31a, 31b in einem Spritzgussverfahren hergestellt ist.

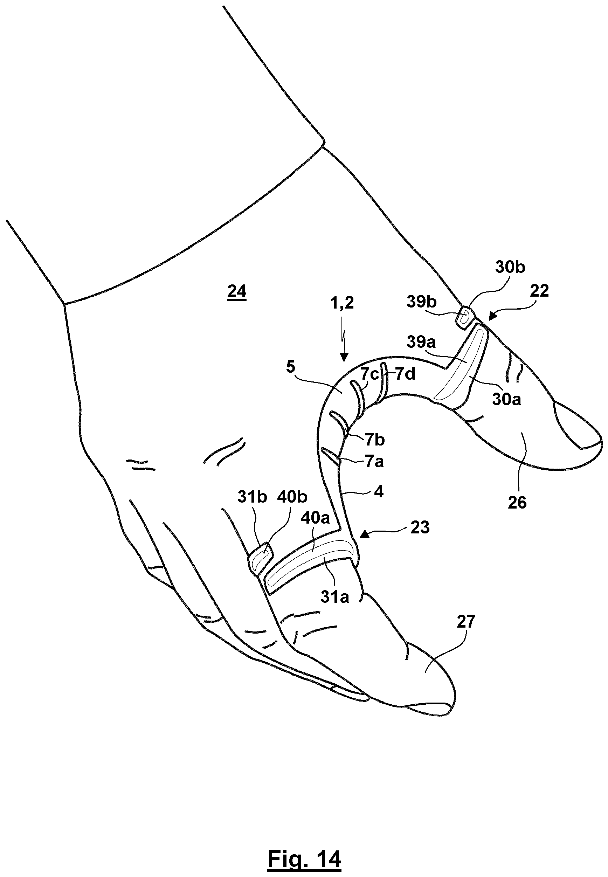

Möglich ist auch, dass die Haltestreifen 30a, 30b, 31a, 31b als Verbundkörper ausgebildet sind. So zeigt **Fig. 14** eine Ausführungsform, in welcher in die Haltestreifen 30a, 30b, 31a, 31b, die beispielsweise aus Kunststoff hergestellt sind, Metallstreifen 39a, 39b, 40a, 40b eingebettet sind, die einerseits eine plastische Verformung zur Anpassung an die Hand 24 ermöglichen und andererseits eine etwaig erforderliche Elastizität gewährleisten.

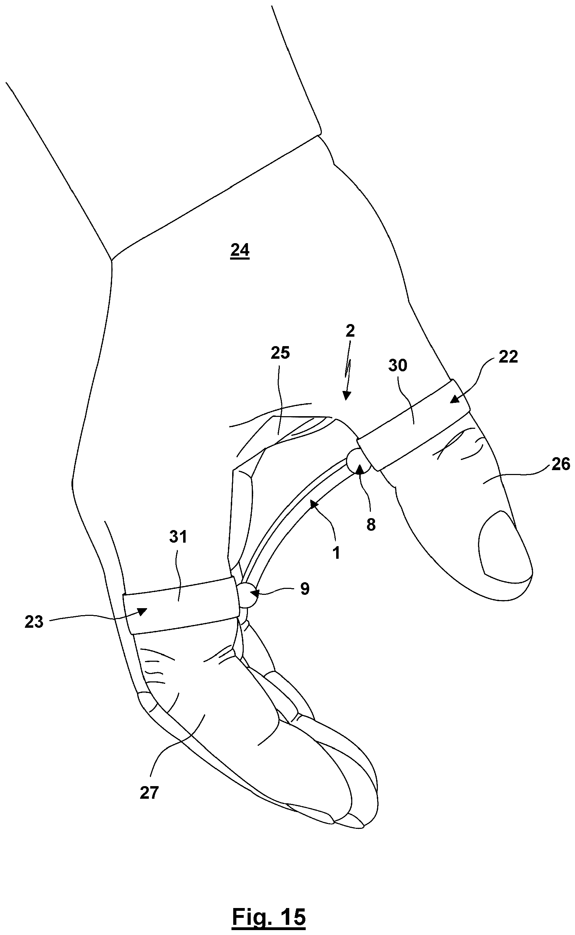

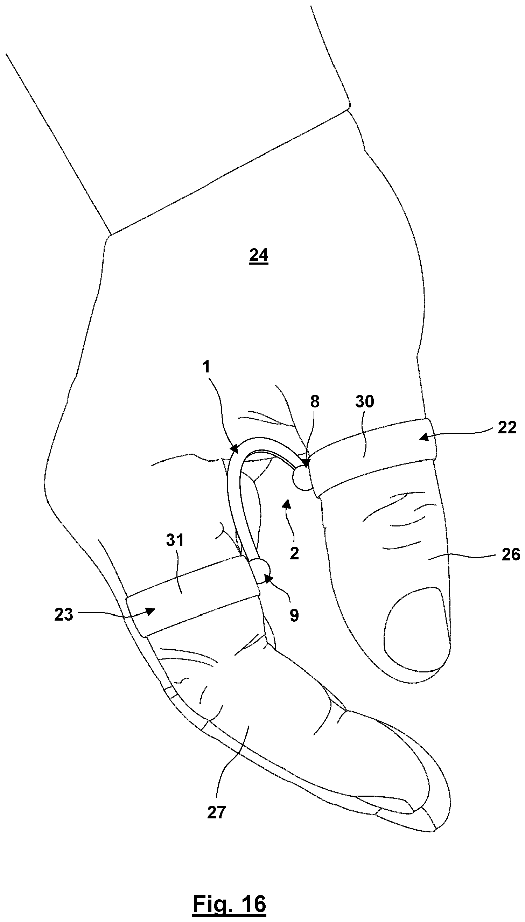

Für die zuvor beschriebenen Ausführungsbeispiele hat der Spreizkörper 1 über seine gesamte Erstreckung an der Hand, nämlich dem Daumen 26, dem Daumenbogen 25 und dem Zeigefinger 27, angelegen. In **Fig. 15** **und** **16** ist eine Ausführungsform dargestellt, bei der dies nicht der Fall ist. Vielmehr stellt für diese Ausführungsform der Spreizkörper 1 eine Art Spange oder Brücke zwischen den Halteeinrichtungen 22, 23 dar, bei der sich der Spreizkörper 1 ohne Anlage an den Daumenbogen 25 zwischen dem Daumen 26 und dem Zeigefinger 27 erstreckt. Der Spreizkörper 1 kann dann eine beliebige Geometrie aufweisen und bspw. als elastischer Bügel oder Biegebalken oder als elastischer Zug- und/oder Druckstange ausgebildet sein,. Hier erfolgt die Verbindung der Halteeinrichtungen 22, 23 mit dem Spreizkörper 1 über Kopplungseinrichtungen 8, 9, die unter Umständen auch eine Art Gelenk ausbilden, womit ein Schwenk-Freiheitsgrad zwischen dem Spreizkörper 1 und den Halteeinrichtungen 22, 23 gewährleistet wird.

Während Fig. 15 die Grundstellung zeigt, in welcher der Spreizkörper 1 nicht elastisch verspannt ist, zeigt Fig. 16 eine elastische Verspannung des Spreizkörpers 1 infolge der durch den Daumen 26 und den Zeigefinger 27 applizierten Kräfte zwecks Annäherung des Daumens 26 an den Zeigefinger 27.

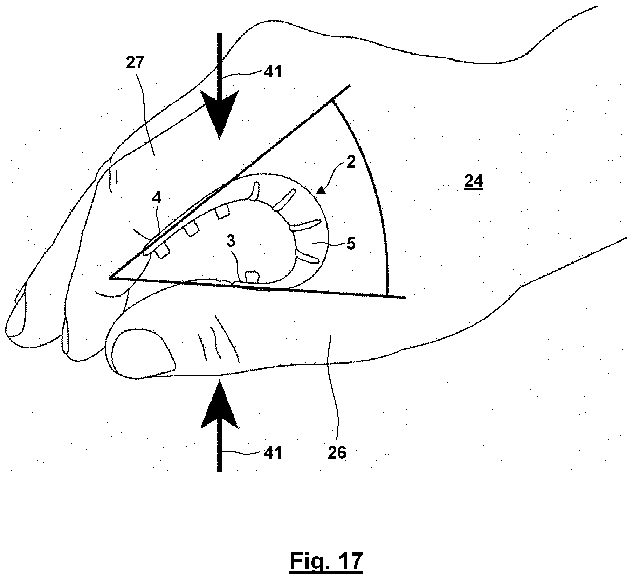

**Fig. 17** zeigt schematisch die bei der Benutzung auf die Daumenorthese 2, hier den Spreizkörper 1, wirkende Kraft 41.

Um die Steifigkeit des Spreizkörpers 1 zu quantifizieren, wird der Endbereich 28 des Seitenschenkels 3 horizontal auf einer Unterlage ausgerichtet. Auf den Endbereich 28 wirkt dann die Kraft 41 ein. Auf der Wirklinie der auf den Endbereich 28 des Seitenschenkels 3 einwirkenden Kraft 41 wirkt dann die entsprechende Gegenkraft 41 auf den Seitenschenkel 4 des Spreizkörpers 1 ein. Erfolgt in dieser Weise eine Messung einerseits der Kraft 41 und andererseits des sich infolge der einwirkenden Kraft 41 verändernden Öffnungswinkels 15, kann auf diese Weise eine Steifigkeit (oder für eine Veränderung des Betrags der Kraft 41 eine Steifigkeitscharakteristik) des Spreizkörpers 1 gemessen werden. Vorzugsweise ist eine Steifigkeit des Spreizkörpers 1 derart gewählt, dass sich mit einer Kraft 41 von 10 Newton eine Änderung des Öffnungswinkels 15 der Endbereiche 28, 29 der Seitenschenkel 3, 4 ergibt, der
a) 45°( ± 20° oder ± 15 oder ± 10° oder ± 5°) oder
b) 30° (± 20° oder ± 15° oder ± 10° oder ± 5°) oder
c) 50° (± 20° oder ± 15° oder ± 19° oder ± 5°)
ergibt, wobei das entsprechende Steifigkeitsverhalten auch gilt für eine Applikation kleinerer Kräfte. Hierbei kann die Steifigkeitscharakteristik der applizierten Kraft über dem Öffnungswinkel 15 ein lineares Verhalten oder auch ein beliebiges nichtlineares Verhalten aufweisen.

Vorzugsweise besteht der Spreizkörper 1 aus einem thermoplastischen Polyurethan. Hierbei kann der Spreizkörper eine Härte Shore A von 80 (± 20 oder 15 ± oder ± 10 oder ± 5) oder eine Härte Shore A von 90 (± 20 oder 15 ± oder ± 10 oder ± 5) aufweisen. Ebenfalls möglich ist aber auch, dass der Spreizkörper 1 aus einem Polyurethan oder Silikon hergestellt ist.

Die Daumenorthese 2 kann beim täglichen Gebrauch eingesetzt werden, ohne dass diese signifikant die Nutzung der Hand 24 beeinträchtigt. Die Daumenorthese 2 ist leicht abnehmbar und kann leicht gereinigt werden. Möglich ist auch, dass die Länge der Haltestreifen 30, 31 durch Abschneiden bedarfsgerecht angepasst wird. Möglich ist, dass an dem Spreizkörper 1 mehrere Kopplungseinrichtungen 8, 9, insbesondere Pilzköpfe 10 in Richtung der Längserstreckung verteilt oder auch nebeneinander angeordnet sind. Möglich ist beispielsweise, dass Kopplungseinrichtungen 8, 9 bzw. Pilzköpfe 10 in der Art von Doppelreihen an dem Spreizkörper 1 vorgesehen sind. Überschüssige Kopplungseinrichtungen 8, 9, insbesondere Pilzköpfe 10, können auch abgetrennt werden. Abweichend zu dem Einsatz von einem Haltestreifen oder zwei Haltestreifen, deren Längserstreckung ungefähr dem Umfang des Daumens 26 bzw. des Zeigefingers 27 entspricht, kann auch ein längerer Haltestreifen eingesetzt werden, der sich dann in der Art einer Spirale um den Daumen 26 bzw. den Zeigefinger 27 erstreckt.

Die erfindungsgemäße Daumenorthese 2 kann auf besonders einfache Weise angelegt werden und wieder abgelegt werden und ist platzsparend sowohl im getragenen Zustand als auch bei der Bevorratung. Durch die Anpassbarkeit kann die Zahl der erforderlichen Modelle für die erforderliche Spanne von Handgrößen reduziert werden. Möglich ist auch, dass dieselbe Daumenorthese für das Tragen an der rechten Hand sowie an der linken Hand eingesetzt werden kann.

### BEZUGSZEICHENLISTE

- 1: Spreizkörper
- 2: Daumenorthese
- 3: Seitenschenkel
- 4: Seitenschenkel
- 5: Grundschenkel
- 6: Führungselement
- 7: Führungsrippe
- 8: Kopplungseinrichtung
- 9: Kopplungseinrichtung
- 10: Pilzkopf
- 11: Solltrennstelle
- 12: Länge erster Grundschenkel (3)
- 13: Länge zweiter Grundschenkel (4)
- 14: Krümmungsradius
- 15: Öffnungswinkel
- 16: Quererstreckung
- 17: Quererstreckung
- 18: Quererstreckung
- 19: Krümmungsradius
- 20: Krümmungsradius
- 21: Krümmungsradius
- 22: Halteeinrichtung
- 23: Halteeinrichtung
- 24: Hand
- 25: Daumenbogen
- 26: Daumen
- 27: Zeigefinger
- 28: Endbereich
- 29: Endbereich
- 30: Haltestreifen
- 31: Haltestreifen
- 32: Stift
- 33: Verbindungseinrichtung
- 34: Verbindungseinrichtung
- 35: Klettverschluss
- 36: Klettverschluss
- 37: Lochstreifen
- 38: Lochstreifen
- 39: Metallstreifen
- 40: Metallstreifen
- 41: Kraft
- 33: Verbindungseinrichtung
- 34: Verbindungseinrichtung
- 35: Klettverschluss
- 36: Klettverschluss
- 37: Lochstreifen
- 38: Lochstreifen
- 39: Metallstreifen
- 40: Metallstreifen
- 41: Kraft

## Patentansprüche

1. Daumenorthese (2) mit einem Spreizkörper (1), der dem Auseinanderspreizen eines Daumens (26) und eines Zeigefingers (27) dient, **gekennzeichnet durch** eine erste Halteeinrichtung (22), über die ein erster Endbereich (28) des Spreizkörpers (1) an dem Daumen (26) gehalten werden kann, und eine zweite Halteeinrichtung (23), über die ein zweiter Endbereich (29) des Spreizkörpers (1) an dem Zeigefinger (27) gehalten werden kann, wobei die Daumenorthese (2) ausschließlich über die beiden Halteeinrichtungen (22, 23) an der Hand (24) der die Daumenorthese (2) tragenden Person gehalten ist.

2. Daumenorthese (2) nach Anspruch 1, **dadurch gekennzeichnet, dass** der Spreizkörper (1) eine Elastizität derart aufweist, dass der Spreizkörper (1) durch von dem Daumen (26) und dem Zeigefinger (27) aufgebrachte Schließkräfte elastisch verformbar ist, so dass der Abstand der Endbereiche (28, 29) des Spreizkörpers (1) verringert wird.

3. Daumenorthese (2) nach einem der vorhergehenden Ansprüche, **dadurch gekennzeichnet, dass** der Spreizkörper (1) einen gekrümmten Grundschenkel (5) und zwei sich von den Endbereichen (28, 29) des Grundschenkels (5) erstreckende Seitenschenkel (3, 4) aufweist, wobei die Seitenschenkel (3, 4) nach außen divergieren.

4. Daumenorthese (2) nach Anspruch 3, **dadurch gekennzeichnet, dass** die Seitenschenkel (3, 4) einen Öffnungswinkel (15) von 60° ± 20° aufweisen.

5. Daumenorthese (2) nach einem der vorhergehenden Ansprüche, **dadurch gekennzeichnet, dass** der Spreizkörper (1) einen gekrümmten Querschnitt aufweist.

6. Daumenorthese (2) nach einem der vorhergehenden Ansprüche, **dadurch gekennzeichnet, dass** der Spreizkörper (1) aus Kunststoff hergestellt ist.

7. Daumenorthese (2) nach einem der vorhergehenden Ansprüche, **dadurch gekennzeichnet, dass** mindestens eine Halteeinrichtung (22, 23) mindestens einen den Daumen (26) oder den Zeigefinger (27) zumindest teilweise umschließenden Haltestreifen (30, 31) aufweist.

8. Daumenorthese (2) nach Anspruch 7, **dadurch gekennzeichnet, dass** Endbereiche (28, 29) von einem Haltestreifen (30, 31) der Halteeinrichtung (22, 23) oder von zwei Haltestreifen (30, 31) der Halteeinrichtung (22, 23) über eine Verbindungseinrichtung (33, 34) miteinander verbindbar sind.

9. Daumenorthese (2) nach einem der vorhergehenden Ansprüche, **dadurch gekennzeichnet, dass** mindestens eine Halteeinrichtung (22, 23) mit dem Spreizkörper (1) über eine lösbare und/oder einen Freiheitsgrad bereitstellende Kopplungseinrichtung (8, 9) verbunden ist.

10. Daumenorthese (2) nach einem der vorhergehenden Ansprüche, **dadurch gekennzeichnet, dass** der Spreizkörper (1) ein Führungselement (6) für einen mit der Hand (24), die mit der Daumenorthese (2) ausgestattet ist, getragenen oder gegriffenen Gegenstand aufweist.

11. Daumenorthese (2) nach einem der vorhergehenden Ansprüche, **dadurch gekennzeichnet, dass** der Spreizkörper (1)
a) einen gekrümmten Grundschenkel (5) aufweist, wobei der Krümmungsradius 25 mm beträgt und/oder
b) einen ersten Seitenschenkel (3) aufweist, dessen Länge 11 mm beträgt und/oder
c) einen zweiten Seitenschenkel (4) aufweist, dessen Länge 30 mm beträgt und/oder
d) im Bereich des ersten Seitenschenkels (3) im Querschnitt einen Krümmungsradius von 18 mm aufweist und/oder
e) im Bereich des zweiten Seitenschenkels (4) im Querschnitt einen Krümmungsradius von 37 mm aufweist und/oder
f) im Bereich des Grundschenkels (5) im Querschnitt einen Krümmungsradius von 12 mm aufweist,
wobei auch Abweichungen der angegebenen Längen und/oder Radien von ± 50 % oder ± 20 % oder ± 10 % oder ± 5 % möglich sind.

12. Daumenorthese (2) nach einem der vorhergehenden Ansprüche, **dadurch gekennzeichnet, dass** die Länge eines Seitenschenkels (3, 4) des Spreizkörpers (1) und/oder die Größe einer Halteeinrichtung (22, 23) oder die Länge mindestens eines Haltestreifens (30, 31) veränderbar ist.

13. Daumenorthese (2) nach Anspruch 12, **dadurch gekennzeichnet, dass** die Länge durch eine Solltrennstelle (11) des Seitenschenkels (3, 4) des Spreizkörpers (1) oder des Haltestreifens (30, 31) veränderbar ist.

14. Set mit mehreren Daumenorthesen (2) nach einem der Ansprüche 1 bis 13, **dadurch gekennzeichnet, dass** das Set
a) Daumenorthesen (2) für Hände unterschiedlicher Größen aufweist, wozu die Daumenorthesen (2), insbesondere die Spreizkörper (1), unterschiedliche Abmessungen aufweisen, und/oder
b) Daumenorthesen (2) mit Spreizkörpern (1) mit unterschiedlichen Elastizitäten aufweist.

## Claims

1. Thumb orthosis (2) comprising a spreading body (1) which serves for spreading a thumb (26) and a pointer finger (27), **characterized by** a first holding device (22) by which a first end portion (28) of the spreading body (1) can be held at the thumb (26) and a second holding device (23) by which a second end portion (29) of the spreading body (1) can be held at the pointer finger (27), the thumb orthosis (2) being exclusively held by the two holding devices (22, 23) at the hand (24) of the user wearing the thumb orthosis (2).

2. Thumb orthosis (2) of claim 1, **characterized in that** the spreading body (1) comprises an elasticity such that it is possible to elastically deform the spreading body (1) by closing forces applied by the thumb (26) and the pointer finger (27) for reducing the distance of the end portions (28, 29) of the spreading body (1).

3. Thumb orthosis (2) of one of the preceding claims, **characterized in that** the spreading body comprises a curved base leg (5) and two side legs (3, 4) which extend from the end portions (28, 29) of the base leg (5), the side legs (3, 4) diverging in outer direction.

4. Thumb orthosis (2) of claim 3, **characterized in that** the side legs (3, 4) have an opening angle (15) of 60° ±20°.

5. Thumb orthosis (2) of one of the preceding claims, **characterized in that** the spreading body (1) comprises a curved cross-section.

6. Thumb orthosis (2) of one of the preceding claims, **characterized in that** the spreading body (1) is made of plastic.

7. Thumb orthosis (2) of one of the preceding claims, **characterized in that** at least one holding device (22, 23) comprises at least one holding strap (30, 31) which at least partially encloses or surrounds the thumb (26) or the pointer finger (27).

8. Thumb orthosis (2) of claim 7, **characterized in that** the end portions (28, 29) of a holding strap (30, 31) of the holding device (22, 23) or the end portions of two holding straps (30, 31) of the holding device (22, 23) can be connected to each other by a connecting device (33, 34).

9. Thumb orthosis (2) of one of the preceding claims, **characterized in that** at least one holding device (22, 23) is connected to the spreading body (1) by a coupling device (8, 9) which is releasable and/or provides a degree of freedom.

10. Thumb orthosis (2) of one of the preceding claims, **characterized in that** the spreading body (1) comprises a guiding element (6) for an object gripped or carried by the hand (24) which is equipped with the thumb orthosis (2).

11. Thumb orthosis (2) of one of the preceding claims, **characterized in that** the spreading body (1)
a) comprises a curved base leg (5), the radius of curvature being 25 mm and/or
b) comprises a first side leg (3) having a length of 11 mm and/or
c) comprises a second side leg (4) having a length of 30 mm and/or
d) in the region of the first side leg (3) has a cross-section with a radius of curvature of 18 mm and/or
e) in the region of the second side leg (4) has a cross-section with a radius of curvature of 37 mm and/or
f) in the region of the base leg (5) has a cross-section with a radius of curvature of 12 mm,
where also deviations from the given lengths and/or radii of ± 50 % or ± 20 % or ± 10 % or ± 5 % are possible.

12. Thumb orthosis (2) of one of the preceding claims, **characterized in that** it is possible to adjust the length of a side leg (3, 4) of the spreading body (1) and/or the size of a holding device (22, 23) or the length of at least one holding strap (30, 31).

13. Thumb orthosis (2) of claim 12, **characterized in that** it is possible to adjust the length by use of a predetermined separation point (11) of the side leg (3, 4) of the spreading body (1) or of the holding strap (30, 31).

14. Set of a plurality of thumb orthoses (2) of one of claims 1 to 13, **characterized in that** the set
a) comprises thumb orthoses (2) for hands of different sizes, wherein the thumb orthoses (2), in particular the spreading bodies (1), have different dimensions and/or
b) comprises thumb orthoses (2) having spreading bodies (1) with different elasticities.

## Revendications

1. Orthèse de pouce (2) avec un corps d'écartement (1) qui permet l'écartement d'un pouce (26) et d'un index (27), **caractérisé par** un premier dispositif de maintien (22) par l'intermédiaire duquel une première partie d'extrémité (28) du corps d'écartement (1) peut être maintenue sur le pouce (26), et un deuxième dispositif de maintien (23), par l'intermédiaire duquel une deuxième partie d'extrémité (29) du corps d'écartement (1) peut être maintenue sur l'index (27), dans lequel l'orthèse de pouce (2) est maintenue exclusivement par l'intermédiaire des deux dispositifs de maintien (22, 23) sur la main (24) de la personne portant l'orthèse de pouce (2).

2. Orthèse de pouce (2) selon la revendication 1, **caractérisée en ce que** le corps d'écartement (1) présente une élasticité de façon à ce que le corps d'écartement (1) puisse être déformé de manière élastique par des forces de fermeture exercées par le pouce (26) et l'index (27), de sorte que la distance entre les parties d'extrémité (28, 29) du corps d'écartement (1) soit réduite.

3. Orthèse de pouce (2) selon l'une des revendications précédentes, **caractérisée en ce que** le corps d'écartement (1) comprend une branche de base incurvée (5) et deux branches latérales (3, 4) s'étendant à partir des parties d'extrémité (28, 29) de la branche de base (5), dans lequel les branches latérales (3, 4) divergent vers l'extérieur.

4. Orthèse de pouce (2) selon la revendication 3, **caractérisée en ce que** les branches latérales (3, 4) présentent un angle d'ouverture (15) de 60°±20°.

5. Orthèse de pouce (2) selon l'une des revendications précédentes, **caractérisée en ce que** le corps d'écartement (1) présente une section transversale incurvée.

6. Orthèse de pouce (2) selon l'une des revendications précédentes, **caractérisée en ce que** le corps d'écartement (1) est constitué d'une matière plastique.

7. Orthèse de pouce (2) selon l'une des revendications précédentes, **caractérisée en ce qu'**au moins un dispositif de maintien (22, 23) comprend au moins une bande de maintien (30, 31) entourant au moins partiellement le pouce (26) ou l'index (27).

8. Orthèse de pouce (2) selon la revendication 7, **caractérisée en ce que** les parties d'extrémité (28, 29) peuvent être reliées ensemble par une bande de maintien (30, 31) du dispositif de maintien (22, 23) ou par deux brandes de maintien (30, 31) du dispositif de maintien (22, 23), par l'intermédiaire d'un dispositif de liaison (33, 34).

9. Orthèse de pouce (2) selon l'une des revendications précédentes, **caractérisée en ce qu'**au moins un dispositif de maintien (22, 23) est relié avec le corps d'écartement (1) par l'intermédiaire d'un dispositif de couplage (8, 9) amovible et/ou permettant un degré de liberté.

10. Orthèse de pouce (2) selon l'une des revendications précédentes, **caractérisée en ce que** le corps d'écartement (1) comprend un élément de guidage (6) pour un objet porté ou saisi avec la main (24) qui est équipée de l'orthèse de pouce (2).

11. Orthèse de pouce (2) selon l'une des revendications précédentes, **caractérisée en ce que** le corps d'écartement (1)
a) comprend une branche de base incurvée (5), dans lequel le rayon de courbure est de 25 mm et/ou
b) comprend une première branche latérale (3) dont la longueur est de 11 mm et/ou
c) comprend une deuxième branche latérale (4) dont la longueur est de 30 mm et/ou
d) présente, au niveau de la première branche latérale (3), en coupe transversale, un rayon de courbure de 18 mm et/ou
e) présente, au niveau de la deuxième branche latérale (4), en coupe transversale, un rayon de courbure de 37 mm et/ou
f) présente, au niveau de la branche de base (5), en coupe transversale, un rayon de courbure de 12 mm,
dans lequel des écarts de ±50 % ou ±20 % ou ±10 % ou ±5 % par rapport aux longueurs et/ou rayons indiqués sont possibles.

12. Orthèse de pouce (2) selon l'une des revendications précédentes, **caractérisée en ce que** la longueur d'une branche latérale (3, 4) du corps d'écartement (1) et/ou la taille d'un dispositif de maintien (22, 23) ou la longueur d'au moins une bande de maintien (30, 31) est variable.

13. Orthèse de pouce (2) selon la revendication 12, **caractérisée en ce que** la longueur peut être modifiée à l'aide d'un point de rupture prédéterminé (11) de la branche latérale (3, 4) du corps d'écartement (1) ou de la bande de maintien (30, 31).

14. Ensemble avec plusieurs orthèses de pouce (2) selon l'une des revendications 1 à 13, **caractérisé en ce que** l'ensemble
a) comprend des orthèses de pouce (2) pour des mains de différentes tailles, les orthèses de pouce (2), plus particulièrement les corps d'écartement (1), présentant pour cela des dimensions différentes et/ou
b) comprend des orthèses de pouce (2) avec des corps d'écartement (1) présentant des élasticités différentes.
